Europäisches Patentamt

European Patent Office

Office européen des brevets

(19)

(11) Numéro de publication: **0 098 222**
**A1**

(12) # DEMANDE DE BREVET EUROPEEN

(21) Numéro de dépôt: 83401321.1

(22) Date de dépôt: 27.06.83

(51) Int. Cl.³: **A 61 B 5/02**
**G 07 F 17/04**

(30) Priorité: 30.06.82 FR 8211516

(43) Date de publication de la demande:
**11.01.84 Bulletin 84/2**

(84) Etats contractants désignés:
**AT BE CH DE GB IT LI LU NL SE**

(71) Demandeur: **Becquet, Jacqueline**
**55, Septième Avenue**
**F-60260 Lamorlaye(FR)**

(71) Demandeur: **Denis, Hervé**
**16, rue de Condé**
**F-75006 Paris(FR)**

(72) Inventeur: **Becquet, Jacqueline**
**55, Septième Avenue**
**F-60260 Lamorlaye(FR)**

(72) Inventeur: **Denis, Hervé**
**16, rue de Condé**
**F-75006 Paris(FR)**

(74) Mandataire: **Rodhain, Claude**
**Cabinet Claude RODHAIN 30, rue La Boétie**
**F-75008 Paris(FR)**

(54) **Appareil de contrôle du rythme cardiaque à détection pléthysmographique.**

(57) L'invention concerne un appareil de contrôle du rytme cardiaque à détection pléthysmographique, comportant un dispositif de mesure pléthysmographique, une unité de traitement des signaux obtenus et un dispositif de visualisation délivrant une information écrite et/ou graphique.

Le problème technique consiste à éliminer, au maximum, les sources d'erreurs, et, en particulier, à éviter les mouvements de l'utilisateur pendant la mesure.

Selon l'invention, le dispositif de mesure pléthysmographique comporte deux capteurs identiques (32) associés, chacun, à un doigt d'une main et, en ce que l'unité de traitement effectue un traitement de corrélation sur les signaux provenant des capteurs (32) précités afin d'éliminer les signaux parasites.

L'invention s'applique en particulier aux appareils disposés dans des lieux publics.

FIG.3

" Appareil de contrôle du rythme cardiaque à détection pléthysmographique ".

L'invention concerne les appareils de contrôle du rythme cardiaque à détection pléthysmographique, c'est-
à-dire des appareils permettant, sans aucune connaissance médicale, de contrôler son rythme cardiaque par enregistrement du pouls
par un procédé pléthysmographique. Ce procédé consiste à enregistrer le changement de volume d'un organe ou d'un membre sous l'influence des variations de calibre des vaisseaux qui l'irriguent.

Les appareils de ce genre comportent de manière générale un dispositif de mesure pléthysmographique, une
unité de traitement de signaux obtenus et un dispositif de visualisation délivrant une information écrite et/ou graphique.

Ces appareils sont mis à la disposition du
public et ils sont généralement payants, c'est-à-dire commandés
par un monnayeur.

L'utilisateur met un doigt, par exemple l'index de la main gauche, dans un capteur et l'appareil délivre un
enregistrement graphique des implusions enregistrées avec une indication en clair sur le rythme cardiaque de l'utilisateur, en
particulier sur sa régularité.

Les études faites par des spécialistes de cardiologie ont montré que, pour avoir une information valable, il
faut que le sujet reste immobile pendant tout le test. Des mouvements du bras ou de la main sont des causes d'erreur fréquentes
et ou risque dans ce cas de fournir des informations fausses à
l'utilisateur qui peut s'alarmer alors qu'en fait son rythme
cardiaque est correct.

Or, ces appareils peuvent être placés dans
des endroits publics où il est difficile d'obtenir le calme et
l'immobilité de l'utilisateur même pendant quelques instants.

Par ailleurs, l'environnement de ces appareils comporte souvent de nombreuses sources de parasites qui
viennent brouiller les signaux formés par le capteur, ce qui peut
également être à l'origine d'erreur.

Il est donc nécessaire de concevoir un appareil de contrôle du rythme cardiaque qui permette d'effectuer une mesure dans les meilleures conditions, en éliminant au maximum les signaux parasites. Pour cela, il est nécessaire de guider l'utilisateur pour qu'il respecte les conditions d'utilisation.

Par ailleurs, il est obligatoire de délivrer une bonne information à l'utilisateur, cela est important dans ce domaine et il faut éviter en particulier, dans le cas où la mesure ne peut pas se faire de manière correcte,de fournir une information erronée.

Dans l'appareil de contrôle du rythme cardiaque selon l'invention, le dispositif de mesure pléthysmographique comporte deux capteurs semblables associés chacun à une des mains de l'utilisateur et l'unité de traitement effectue une corrélation des signaux des deux capteurs.

Le fait d'effectuer une mesure sur les deux mains de l'utilisateur permet de l'immobiliser et de le calmer en captant son attention sur l'appareil; cela élimine donc de nombreuses possibilités d'erreur de mesure. Par ailleurs, le traitement de corrélation effectué sur les signaux provenant des deux capteurs qui sont strictement semblables permet d'éliminer la majorité des signaux parasites.

Selon une autre caractéristique de l'invention, l'appareil comporte un dispositif capteur de position pour chacun des doigts dont on mesure le pouls et les signaux émis par ces capteurs sont envoyés à l'unité de traitement qui, en cas de mauvais positionnement, commande l'affichage sur le dispositif de visualisation d'instructions guidant l'utilisateur en vue de corriger la position de ses doigts.

Cette disposition permet d'obtenir les meilleures conditions de mesure et par suite de fournir une information valable à l'utilisateur.

Selon au autre caractéristique de l'invention, l'appareil comporte un capteur de proximité et les signaux émis par ledit capteur de proximité sont envoyés à l'unité de traitement qui, lorsqu'une personne s'approche de l'appareil, commande l'affichage d'instructions pour l'utilisation de l'appareil sur le dispositif de visualisation.

De cette manière, l'utilisateur est guidé entièrement, ce qui permet de faire la mesure sur une personne calme, c'est-à-dire d'obtenir une information sur le rythme cardiaque normal de l'utilisateur.

Avantageusement, l'unité de traitement effectue un calcul sur les premières impulsions mesurées afin de déterminer une " fenêtre" d'apparition prévue pour les impulsions suivantes et si une impulsion ne se produit pas dans la fenêtre prévue, elle est créée par l'unité de traitement. Par contre, si deux impulsions manquent, l'unité de traitement bloque la délivrance de l'information écrite et/ou graphique, commande l'affichage de l'impossibilité de la mesure et, éventuellement, d'une instruction de récupération de l'argent versé.

Le fait de ne délivrer une information écrite et/ou graphique que si la mesure a pu être effectuée de manière sûre est important car l'utilisateur n'aura pas d'information erronée qui risque de l'alarmer sans raison; on lui indique simplement que la mesure n'a pas été faite.

D'autres caractéristiques et avantages de l'invention apparaîtront au cours de la description qui suit, faite à titre illustratif et nullement limitatif en se référant aux dessins ci-annexés sur lesquels :

- la figure 1 est une vue en perspective d'un appareil de contrôle du rythme cardiaque selon l'invention ;

- la figure 2 est un schéma montrant les différents éléments constitutifs de l'appareil, et ;

- la figure 3 représente un mode de réalisation des dispositifs capteurs de position.

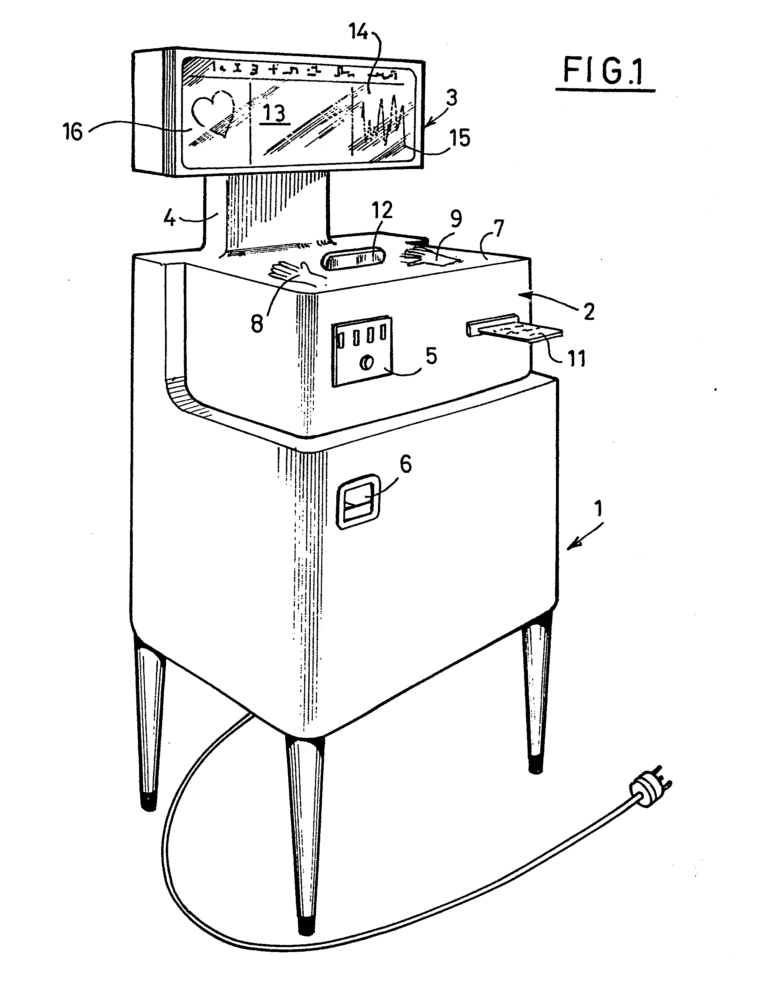

La figure 1 est une vue externe générale d'un appareil de contrôle du rythme cardiaque conforme à la présente invention.

- 4 -                    0098222

Il comporte un corps principal 1 sur lequel est disposé un caisson 2 et qui est surmonté d'uné console de visualisation 3. Cette console 3 est reliée au corps 1 par une colonne 4. Cet appareil est à paiement et comporte, à cet effet, un monnayeur 5 de type connu et un dispositif de retour de la monnaie 6.

La face supérieure 7 du caisson 2 constitue une platine sur laquelle sont dessinées deux mains 8 et 9 facilitant le positionnement des mains de l'utilisateur. Un capteur (non représenté) est disposé sous l'emplacement d'un des doigts de chaque main. Ces deux capteurs sont de même type et strictement semblables, ils peuvent être de tout type connu pour réaliser un enregistrement pléthysmographique, tels que des capteurs optiques à infrarouge, des capteurs à ultrasons, etc... Dans le cas où on utilise des capteurs à infrarouges, le contact avec le doigt peut se faire au travers d'une lentille spéciale faisant légèrement saillie sur la platine 7.

Le dispositif de visualisation est constitué de trois éléments ; le premier est un dispositif 11 distribuant une information à l'utilisateur, constituée par exemple par un enregistrement graphique d'impulsions portant les résultats de la mesure (fréquence et régularité du rythme cardiaque). Sur la platine 7, on prévoit un dispositif d'affichage 12, constitué par exemple, par un afficheur numérique à 7 segments à 3 chiffres et indiquant la fréquence du rythme cardiaque.

Enfin, la console 3 comporte un dispositif central 13 d'affichage de messages à l'attention de l'utilisateur, qui peut être entouré d'inscriptions et de motifs graphiques constituant une animation destinée à attirer l'attention du public. Ces motifs et ces inscriptions sont, de préférence, lumineux et leur éclairage, continu ou clignotant, est commandé par une unité de traitement électronique disposée dans le caisson 2.

Le dispositif central 13 d'affichage de messages peut être constitué par tout dispositif connu tel qu'un écran de télévision ou un magnétoscope. Avantageusement, un microprocesseur, qui sera décrit plus loin, initie des messages d'autoguidage suivant un mode bien connu de chaînage de caractères.

Selon un mode de réalisation de l'invention, ce dispositif d'affichage 13 est constitué par un écran dépoli recevant des images fournies par projecteur de diapositives disposé dans le caisson. Ces images sont transmises par un système optique à réflexion de type connu utilisant la colonne 4 pour le passage du faisceau. Avantageusement, les diapositives à projeter sont disposées à plat sur le bord d'un disque circulaire entraîné par un moteur pas-à-pas commandé par l'unité de traitement.

Les images envoyées sur l'écran 13 peuvent également être obtenues à partir d'enregistrements de photographies électroniques telles que celles annoncées récemment par la presse.

Le dispositif 11 fournissant une information écrite est avantageusement constitué par une imprimante semigraphique alimentée par un chargeur de bande de papier préplié. Comme on le verra plus bas, cette information écrite n'est délivrée qu'à la fin de la mesure.

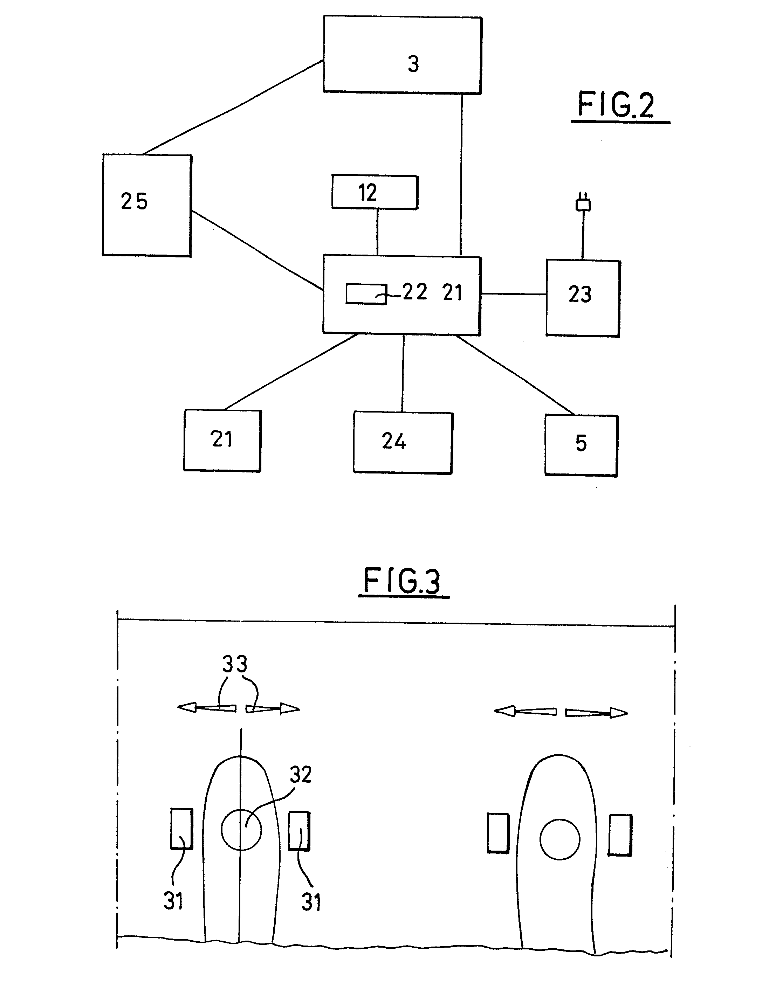

La figure 2 représente schématiquement le dispositif de commande de l'appareil selon l'invention. Il comporte essentiellement une unité de traitement 21 qui peut être une logique câblée ou un microprocesseur comprenant une mémoire 22.

Une alimentation 23 branchée sur le secteur électrique fournit l'énergie électrique nécessaire à tous les organes du dispositif de commande. Ce dernier reçoit des informations provenant du monnayeur 5 et des capteurs 24 et il commande le fonctionnement du dispositif 11 fournissant une information écrite, des afficheurs 12, de la console 3 et du dispositif de projection d'images 25, constitué par exemple comme indiqué plus haut par un projecteur de diapositives associé à un disque support de diapositives.

Conformément à l'invention, les deux capteurs pléthysmographiques sont strictement identiques. Ils sont branchés en parallèle et la mesure est faite simultanément sur les deux mains. Le microprocesseur effectue un traitement de corrélation sur les deux signaux obtenus de manière à éliminer les signaux parasites.

Selon l'invention, on prévoit des capteurs de position qui sont associés à chacun des capteurs pléthysmographiques et dont les signaux sont envoyés au microprocesseur qui, lorsqu'un doigt est mal positionné, commande l'affichage d'instructions demandant à l'utilisateur de bien placer ses mains. Ces capteurs de position peuvent être constitués, comme représenté figure 3, par deux capteurs 31 fonctionnant en tout ou rien, disposés de part et d'autre de chaque capteur pléthysmographique 32. La détection du bon positionnement des doigts peut également être obtenue grâce aux capteurs pléthysmographiques 32 eux-mêmes, en particulier lorsqu'il s'agit de capteurs optiques.

L'affichage d'instructions pour le bon positionnement des mains peut être complété par des symboles lumineux tels que les flèches 33 représentées figure 3.

L'appareil de contrôle du rythme cardiaque, selon l'invention, peut également comporter un capteur de proximité (non représenté) qui détecte la présence d'une personne près de l'appareil, dans une zone de 1 mètre par exemple. Ce capteur peut être de tout type connu ; à titre d'exemple, on peut utiliser des dispositifs à ultrasons analogues à ceux utilisés pour le réglage automatique de la distance dans les appareils photographiques ; on peut également utiliser le dispositif connu sous le nom de "Space detector" comportant un émetteur radio disposé, avec son antenne, à l'arrière de l'appareil et une antenne de réception noyée dans l'avant de l'appareil, la détection étant faite par couplage d'antenne.

Conformément à l'invention, l'unité de traitement, dans l'exemple décrit le programme enregistré dans la mémoire 22, effectue un traitement sur les signaux correspondant aux premières pulsations détectées (par exemple 4 ou 5) et détermine, par calcul, une "fenêtre" d'apparition des signaux suivants, c'est-à-dire un intervalle de temps pendant lequel le signal doit survenir. On réalise ensuite une "poursuite" des signaux suivants consistant à contrôler l'apparition des signaux suivants dans les fenêtres précédemment calculées. Si un signal n'apparaît pas, il est recréé par le programme. S'il en manque encore un autre, l'unité de traitement arrête la mesure, elle bloque la délivrance de l'information écrite du dispositif 11 , elle informe l'utilisateur par l'intermédiaire de l'écran 13 que la mesure n'a pu être effectuée et elle rend l'argent versé par l'utilisateur. A cet effet, le monnayeur 5 est du type à trois voies permettant le rejet des pièces non conformes, le rejet des pièces lorsque la mesure ne peut être effectuée et l'acceptation des pièces en fin de mesure.

Cette méthode de traitement des signaux permet d'obtenir une information sûre. En effet, la disparition d'une impulsion peut être dûe à un mouvement de l'utilisateur; par contre, s'il en manque plus, il peut s'agir d'une anomalie cardiaque mais aussi d'erreurs de manipulation de l'utilisateur et il ne faut pas, dans ce cas, délivrer une information fausse qui pourrait créer une crainte non justifiée chez l'utilisateur.

Selon une caractéristique de l'invention, le dispositif de visualisation 13 est utilisé pour la diffusion de messages publicitaires lorsque l'appareil n'est pas utilisé. Cette diffusion est mise en route par l'arrêt de la mesure et elle est interrompue par l'arrivée d'une personne près de l'appareil. Ceci est obtenu grâce au capteur de proximité cité plus haut dont les signaux traités par le microprocesseur commandent l'arrêt de la diffusion de messages publicitaires.

Le fonctionnement de l'appareil qui vient d'être décrit est le suivant :

S'il n'y a pas de mesure et si personne n'est à proximité, un message lumineux d'appel est affiché sur la console, par exemple sur la partie supérieure 14 et les motifs graphiques lumineux, tels qu'un coeur schématisé et des courbes de pulsation sont affichés en 15 et 16, de part et d'autre de l'écran 13. Ces affichages d'appel sont avantageusement clignotants. L'écran 13 diffuse des messages publicitaires provenant du dispositif de projection de diapositives.

Lorsqu'une personne s'approche de l'appareil, sa présence est détectée par le capteur de proximité, ce qui entraîne la projection, sur l'écran 13, d'une première instruction indiquant, par exemple, le nombre de pièces à introduire. Au fur et à mesure de l'introduction des pièces, le nombre des pièces à introduire est modifié. En cas de mauvais fonctionnement, une instruction d'actionner le bouton de déblocage du monnayeur apparaît ; ceci peut être accompagné du clignotement d'un voyant situé près de ce bouton.

Si le paiement s'est bien effectué, l'écran projette un message pour le positionnement des mains et, afin de calmer le client, on peut arrêter le clignotement des affichages d'appel 14, 15 et 16 qui restent allumés en permanence. Si les doigts sont mal positionnés, les capteurs de position 31 associés aux capteurs pléthysmographiques 32 envoient un signal correspondant au microprocesseur, ce qui commande la projection d'une instruction de mise en place des mains avec clignotement de la flèche 33 appropriée.

Si la mesure peut être effectuée, en particulier si l'on ne détecte pas l'absence de plus d'une impulsion, l'écran affiche une instruction pour retirer l'enregistrement écrit, les afficheurs 12 indiquent le rythme de la pulsation et l'imprimante 11 délivre un ticket à l'utilisateur.

Si la mesure n'est pas correcte, il y a diffusion d'un message indiquant l'impossibilité de faire la mesure et demandant à l'utilisateur de reprendre son argent.

Lorsque l'utilisateur a quitté l'appareil, le détecteur de proximité commande la diffusion des messages publi- taires et le retour au clignotement des affichages d'appel.

Tous les messages d'instructions comprennent avantageusement une partie écrite et un symbole graphique significatif (vue d'un monnayeur, vue d'une main, etc...) ; de plus, ils sont accompagnés du clignotement de voyants appropriés indiquant l'élément à actionner 8 tels que les flèches 33. Ceci permet l'utilisation par tout public non averti, même étranger.

L'appareil selon l'invention est réalisé de manière modulaire, en particulier en ce qui concerne le dispositif électronique de commande dont les différents modules sont enfichables ; cela permet une maintenance rapide, en particulier, on doit pouvoir charger rapidement le support de diapositives pour changer les messages d'instructions et de publicité. Le programme de fonctionnement peut être modifié facilement par remplacement de la mémoire 22. En particulier, les messages et les enregistrements fournis aux utilisateurs doivent pouvoir être changés facilement en fonction de la langue du pays d'utilisation.

La description ci-dessus n'a été fournie qu'à titre d'exemple nullement limitatif et il est évident que l'on peut y apporter des modifications ou variantes sans pour autant sortir du cadre de la présente invention.

REVENDICATIONS

1. Appareil de contrôle du rythme cardiaque à détection pléthysmographique, comportant un dispositif de mesure pléthysmographique, une unité de traitement des signaux obtenus et un dispositif de visualisation délivrant une information écrite et/ou graphique, caractérisé en ce que le dispositif de mesure pléthysmographique comporte deux capteurs identiques (32) associés, chacun, à un doigt d'une main et, en ce que l'unité de traitement (21) effectue un traitement de corrélation sur les signaux provenant des capteurs (32) précités afin d'éliminer les signaux parasites.

2. Appareil selon la revendication 1, caractérisé en ce qu'il comporte un dispositif capteur de position (31) pour chacun des doigts dont on mesure le pouls et en ce que les signaux fournis par ces capteurs de position (31) sont envoyés à l'unité de traitement (21) qui, en cas de mauvais positionnement, commande l'affichage sur le dispositif de visualisation (13) d'instructions guidant l'utilisateur en vue de corriger la position de ses doigts.

3. Appareil selon l'une quelconque des revendications 1 et 2, caractérisé en ce qu'il comporte un capteur de proximité dont les signaux sont envoyés à l'unité de traitement (21) qui, lorsqu'une personne s'approche de l'appareil, commande l'affichage d'instructions pour l'utilisation de l'appareil sur le dispositif de visualisation (13) .

4. Appareil selon l'une quelconque des revendications 1 à 3, caractérisé en ce que l'unité de traitement (21) effectue un calcul sur les premières impulsions détectées, afin de déterminer une fenêtre d'apparition prévue pour les impulsions suivantes et elle réalise un processus de poursuite en surveillant l'apparition desdites impulsions suivantes.

5. Appareil selon la revendication 4, caractérisé en ce que, en cas d'absence d'une impulsion pendant le processus de poursuite, l'unité de traitement (21) recrée l'impulsion manquante et en ce que, en cas d'absence d'une autre impulsion, l'unité de traitement (21) stoppe la mesure, bloque la délivrance d'une information écrite et commande l'affichage d'une instruction d'impossibilité de mesure et, éventuellement, de récupération de l'argent.

6. Appareil selon la revendication 1, caractérisé en ce que les capteurs pléthysmographiques (32) sont du type optique à infrarouges.

7. Appareil selon la revendication 2, caractérisé en ce que les capteurs de position sont constitués, chacun, par deux capteurs (31) fonctionnant par tout ou rien et disposés de part et d'autre du capteur pléthysmographique (32).

8. Appareil selon la revendication 2, caractérisé en ce que les capteurs de position sont constitués par le capteur pléthysmographique (32) lui-même.

9. Appareil selon la revendication 3, caractérisé en ce que le capteur de proximité commande la diffusion de messages publicitaires par le dispositif de visualisation (13) lorsque l'appareil n'est pas utilisé.

10. Appareil selon la revendication 3, caractérisé en ce que le détecteur de proximité est un appareil à ultrasons.

11. Appareil selon la revendication 3, caractérisé en ce que le détecteur de proximité est un dispositif à émetteur radio avec couplage d'antenne de réception.

12. Appareil selon la revendication 1, caractérisé en ce que l'unité de traitement est constitué par un microprocesseur (21) comportant une mémoire (22) de microprogrammation.

13. Appareil selon la revendication 1, caractérisé en ce que le dispositif de visualisation comporte un écran (13) recevant des images fournies par un projecteur de diapositives.

14. Appareil selon la revendication 1, caractérisé en ce que le dispositif de visualisation comporte un écran de télévision.

FIG.1

2/2

3

FIG.2

25

12

22 21

23

21

24

5

FIG.3

33

32

31          31

0098222

Office européen
des brevets

**RAPPORT DE RECHERCHE EUROPEENNE**

Numéro de la demande

EP 83 40 1321

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | MEDICAL & BIOLOGICAL ENGINEERING & COMPUTING, vol. 15, no. 3, mai 1977, pages 228-231, USA L.A. GEDDES et al.: "Multifunction transducer for obtaining digital volume pulse, skin-resistance response and electrocardiogram" * I "Introduction", II "The transducer" * | 1 | A 61 B 5/02 G 07 F 17/04 |
| | --- | | |
| A | BE-A- 789 647 (INTERNATIONAL CONTACTS INVENTORS) * En entier * | 1,2,6, 8,14 | |
| | --- | | |
| A | ELEKTRONIK, vol. 24, no. 9, 1975, pages 100-102, München, DE. T. DVORAK et al.: "Ein Cardiotachometer zur kontinuierlichen Erfassung der Herzrate" * En entier * | 1,4,6 | |
| | --- | | **DOMAINES TECHNIQUES RECHERCHES (Int Cl. ³)** |
| A | US-A-3 835 838 (INTERNATIONAL CONTACTS INVENTORS) * Colonne 2, ligne 53 - colonne 4, ligne 26; figures 1-3 * | 1,14 | A 61 B 5/02 A 61 B 5/06 G 07 F 17/04 |
| | --- | | |
| A | CH-A- 621 056 (G. HIRSCH) * Page 3, colonne de gauche, ligne 9 - page 3, colonne de droite, ligne 39; figures 1-4 * | 6,8,12 | |
| | ---     -/- | | |

Le présent rapport de recherche a été établi pour toutes les revendications

| Lieu de la recherche LA HAYE | Date d'achèvement de la recherche 01-09-1983 | Examinateur ZILLIOX J.M. |
|---|---|---|

**Office européen des brevets**

# RAPPORT DE RECHERCHE EUROPEENNE

Numéro de la demande

EP 83 40 1321

| DOCUMENTS CONSIDERES COMME PERTINENTS | | Page 2 |

| Catégorie | Citation du document avec indication, en cas de besoin, des parties pertinentes | Revendication concernée | CLASSEMENT DE LA DEMANDE (Int. Cl. ³) |
|---|---|---|---|
| A | DE-A-3 040 524 (MICROTEC ELECTRONIC GmbH) * Page 8, ligne 9 - page 9, ligne 26; page 17, ligne 3 - page 18, ligne 24; figures 1-7 * | 4 | |
| | --- | | |
| A | CH-A- 286 667 (R. KOECHLIN) * Page 1, ligne 52 - page 2, ligne 3; page 2, lignes 42-48; page 2, lignes 82-94; figure 1 * | 1 | |
| | --- | | |
| A | US-A-3 841 314 (R.E. PAGE) * Colonne 3, ligne 25 - colonne 5, ligne 63; figures 1-4 * | 1 | |
| | --- | | |
| A | FR-A-1 268 877 (E. JAEGER) * Page 1, colonne de gauche, lignes 1-15; page 1, colonne de droite, lignes 3-15; page 2, colonne de gauche, lignes 20-35; page 2, colonne de droite, ligne 53 - page 4, colonne de gauche, ligne 26; page 5, colonne de gauche, ligne 40 - page 5, colonne de droite, ligne 6; figures 1-6 * | 1 | DOMAINES TECHNIQUES RECHERCHES (Int. Cl. ³) |
| | ----- | | |

Le présent rapport de recherche a été établi pour toutes les revendications

| Lieu de la recherche | Date d'achèvement de la recherche | Examinateur |
|---|---|---|
| LA HAYE | 01-09-1983 | ZILLIOX J.M. |